# EUROPÄISCHE PATENTSCHRIFT

(11) **EP 2 335 826 B1**
(45) Veröffentlichungstag und Bekanntmachung des Hinweises auf die Patenterteilung: **14.05.2014**
(21) Anmeldenummer: 10405190.9
(22) Anmeldetag: 12.10.2010
(51) Int. Cl.: B01L 3/00, A61B 10/00, G01N 35/00

(54) **Indentifizierbarer Behälter**
Identifiable container
Récipient identifiable

(30) Priorität: 30.11.2009 CH 18332009
(43) Veröffentlichungstag der Anmeldung: 22.06.2011
(73) Patentinhaber: Berlinger & Co., 9608 Ganterschwil (CH)
(72) Erfinder: Oertli, Christian, 8620 Wetzikon (CH); Berlinger, Andrea, 9608 Ganterschwil (CH)
(74) Vertreter: Quehl, Horst Max

(56) Entgegenhaltungen:
- EP-A1- 1 832 878
- CN-Y- 2 707 659
- DE-A1- 4 306 563
- DE-A1- 4 318 311
- FR-A1- 2 861 702
- US-A- 5 777 303
- US-A1- 2006 158 332
- US-A1- 2008 063 563
- US-B2- 7 258 840

## Beschreibung

Die Erfindung betrifft einen identifizierbarer Behälter für eine fälschungssichere Aufbewahrung von Substanzen oder Objekten, mit einer Füllöffnung und einer dieser zugeordneten Verschlussvorrichtung, wobei die Verschlussvorrichtung Verriegelungsmittel aufweist durch die sie zerstörungsfrei unlösbar am Behälter befestigt ist und mit einer ein- und auslesbaren elektronischen Speichereinheit.

Behälter dieser Art sind bekannt durch die US 7,258,840 und die EP 0 875 292. Bei ihnen besteht die elektronische Speichereinheit aus einem Transponder, der mit dem Behälter fest verbunden ist. Das Ein- oder Auslesen von Daten erfolgt somit kontaktlos durch drahtlose Signalübertragung. Ein weiterer Behälter mit einer elektronischen Speichereinheit ist bekannt durch die FR 2861702. Diese ist in der Verschlussvorrichtung eingeschlossen aber ebenfalls nur kontaktlos ein- und auslesbar. Im betreffenden Sachgebiet sind durch die DE 4306563, die US 5777303 und die US 2008/0063563 auch elektronische Speichereinheiten bekannt, die nur über mechanische Kontaktmittel ein- und auslesbar sind. Sie sind jedoch entweder in einem Behälterboden oder in einem Teil angeordnet, das mit dem Behälterboden zusammenwirkt. Behälter mit einer durch mechanischen Kontakt ein-und auslesbaren Speichereinheiten sind ausserdem durch die US 2006/ 0158332, die CN 2707659 Y und die EP 1832878 bekannt, jedoch sind auch diese nicht fälschungssicher im Behälterverschluss angeordnet.

Der Erfindung liegt die Aufgabe zugrunde, einen Behälter der eingangs genannten Art zu finden, der bei kostengünstiger Herstellbarkeit eine sichere Aufbewahrung und Identifikation seines Inhaltes gewährleistet, so dass er besonders für die Aufbewahrung von Urin- oder Blutproben einer Dopingkontrolle geeignet ist.

Die Lösung der genannten Aufgabe erfolgt erfindungsgemäss aufgrund der kennzeichnenden Merkmale des Patentanspruchs 1. Vorteilhafte Ausgestaltungen der Erfindung sind Gegenstand der abhängigen Patentansprüche und der folgenden Beschreibung anhand der Zeichnungen zu entnehmen.

Es zeigt:
Fig.1 einen Axialschnitt durch einen erfindungsgemässen Behälter in verschlossenem Zustand,
Fig.2 eine Seitenansicht des Behälters nach Fig.1 ohne seine Schutzkappe,
Fig.3 eine perspektivische Darstellung der Schutzkappe des Behälters nach Fig.1,
Fig.4 eine perspektivische Darstellung eines für die zerstörungsfrei unlösbare Verbindung der Schutzkappe mit dem Behälter vorgesehenen Sperrnockenring,
Fig.5 eine perspektivische Darstellung eines mit dem Sperrnockenring zusammenwirkenden Federzungenringes,
Fig.6 eine perspektivische Darstellung eines Abdeckringes, der eine ungewollte Verriegelung der Schutzkappe am Behälter verhindert,
Fig.7 eine Aufsicht auf den Behälter nach Fig.2 in perspektivischer Darstellung und
Fig.8 eine separate perspektivische Darstellung der in Fig.7 gezeigten, elektronischen Speichereinheit.

Der Behälter 1 des dargestellten Ausführungsbeispiels ist flaschenförmig als Glaskörper ausgebildet und hat einen mit einem Aussengewinde 2 versehenen Füllstutzen 3, auf den eine Schutzkappe 4 aufgeschraubt ist.

Für eine zugriffssichere Aufbewahrung, z.B. von Urin- oder Blutproben einer Dopingkontrolle, ist die Schutzkappe 4 in vollständig aufgeschraubter Position zerstörungsfrei unlösbar mit dem Behälter 1 verbunden. Damit sich diese Art der Verbindung beim Aufschrauben der Schutzkappe 4 selbsttätig ergibt, sind zwischen beiden Verrastungsmittel vorgesehen, wie es durch die DE 43 18 311 an sich bekannt ist. Diese Verrastungsmittel bestehen aus z.B. vier Rastnocken 5, 6, die gleichmässig am Umfang verteilt einerseits an einem umlaufenden, an den Füllstutzen 3 angrenzenden Schulterbereich 7 des Behälters 1 vorgesehen sind und andererseits aus z.B. sieben Rastnocken 8 an einem in der Schutzkappe 4 verdrehfest und axial beweglich eingeschlossenen Sperrnockenring 9.

Die verdrehfeste und axiale Beweglichkeit des Sperrnockenringes 9 ergibt sich durch mehrere vom Sperrnockenring 9 radial abstehende Führungsnocken 10, die jeweils in einer achsparallel verlaufenden Führungsrille 11 im Kappenkragen 12 der Schutzkappe 4 geführt sind. Ein axial wirkender Federring 13 mit z.B. vier ausgestanzten Federzungen 14 hält den Sperrnockenring 8 sicher in seiner Sperrposition.

Damit diese Verrastungsmittel 5,6,7 nicht unbeabsichtigt in zerstörungsfrei unlösbaren gegenseitigen Eingriff gelangen können, d.h. bevor der Behälter 1 gebraucht wird, ist zwischen dem Sperrnockenring 9 und dem Rastnocken 5,6 aufweisenden Schulterbereich 7 des Behälters 1 ein Abdeckring 15 eingesetzt, der somit vor Verschliessen des Behälters zu entfernen ist.

Sowohl der Behälter 1 als auch seine Schutzkappe 4 bestehen aus einem unverformbaren und z.B. bei Schlageinwirkung zerbrechlichen Material, wie z.B. aus Glas oder einem Kunststoff wie z.B. Polycarbonat mit vergleichbaren Eigenschaften, so dass ein missbräuchliches Öffnen der Schutzkappe 4 durch Einschneiden oder Verformung z.B. unter Wärmeeinwirkung ohne sichtbare Beschädigung unmöglich ist.

Da ein solches unter Schlageinwirkung zerbrechliches Material aufgrund seines Härtegrades in gegenseitigem Kontakt keine optimale, d.h. flüssigkeits- und gasdichte Abdichtung gewährleistet, umschliesst die Schutzkappe 4 weiterhin einen Innenverschluss 16 aus einem weichelastischen Material. Dieser liegt mit einer Schulterfläche 18 an der Stirnfläche 17 des Füllstutzens 3 an und ein umlaufende Dichtlippen 19 aufweisender Hohlzapfen 20 des Innenverschlusses 16 wird beim Aufschrauben der Schutzkappe 4 dichtend in den Füllstutzen 3 eingedrückt.

Auf der dem Füllstutzen 3 abgekehrten Seite bildet der Innenverschluss 16 einen zylindrischen Aufnahmeraum 21 für eine elektronische Speichereinheit 22, dessen Aussenrand 23 ringförmig nebeneinander angeordnete Fortsätze 24 aufweist, über die sich der Innenverschluss 16 bei fest aufgeschraubter Schutzkappe 4 an der endseitigen Schutzkappenwand 25 elastisch abstützt. Auf diese Weise ist der Aufnahmeraum 21 des Innenverschlusses 16 gegenüber dem Behälteraufnahmeraum 26 bzw. dessen Inhalt dicht abgeschlossen und eine in ihm eingeschlossene, nur über mechanische Kontaktmittel 27 ein- und auslesbare elektronische Speichereinheit 22 ist, ebenso wie der Behälterinhalt, durch die Schutzkappe 4 zugriffssicher und damit fälschungssicher eingeschlossen.

Ein Entleeren des Behälters 1 oder ein Kontakt zum Behälterinhalt und zu der elektronischen Speichereinheit ist somit nur durch Zerstörung der Schutzkappe 4 möglich, derart dass der Eingriff zwischen dem Sperrnockenring 9 und den Rastnocken 5,6 des Behälters 1 gelöst wird.

Damit es zum Entfernen der Schutzkappe 4 nicht erforderlich ist, diese insgesamt zu zerstören, ist im Schulterbereich 28 zwischen dem Kappenkragen 12 und dem übrigen, das Schraubgewinde aufweisenden Teil der Schutzkappe 4, vorzugsweise auf der Innenseite der Schutzkappe 4, als Solltrennstelle eine umlaufende Rille 29 eingeformt. Diese ermöglicht eine saubere Abtrennung des Kappenkragens 12 durch Anwendung einer zum Behälter 1 achsparallelen Presskraft auf die umlaufende Aussenkante 30 des Kappenkragens 12. Vorzugsweise wird diese gleichmässig am gesamten Umfang dieser Aussenkante 30 angewandt, wie es z.B. durch einen ringförmigen, auf diese Kante 30 aufgesetzten, nichtdargestellten Presstempel möglich ist. Dieser kann Bestandteil z. B. einer für diesen Zweck ausgebildeten Schraubpresse sein.

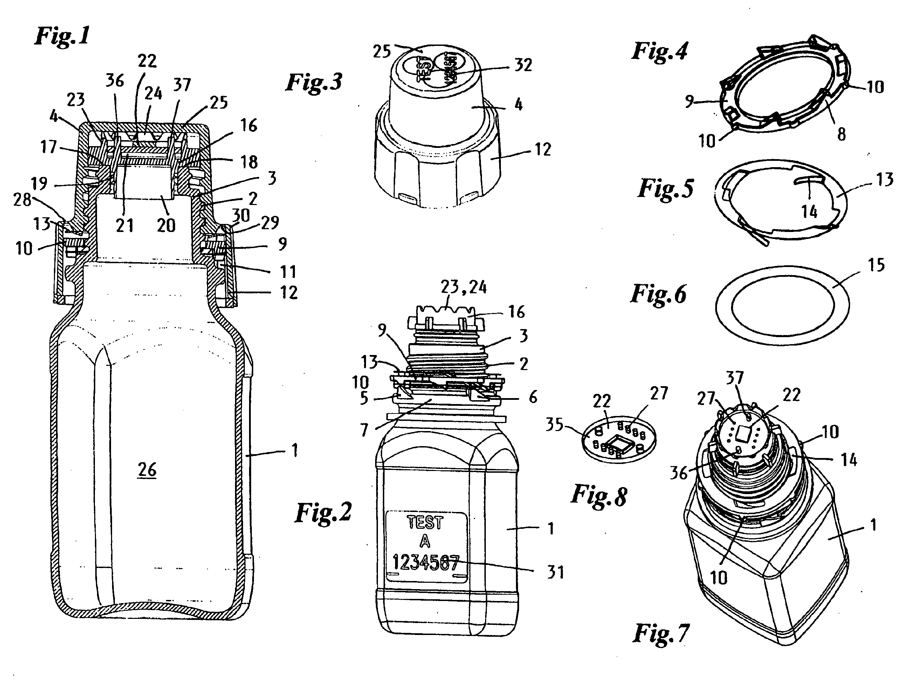

Die Darstellungen der Fig.2 und Fig.7 zeigen den Behälter 1 nach vollständiger Entfernung der Schutzkappe 4. Dabei befindet sich der Innenverschluss 16 noch in dichtendem Eingriff mit dem Füllstutzen 3 des Behälters 1 und die elektronische Speichereinheit 22 befindet sich in einer zum Auslesen von Daten gut geeigneten Position, in der mehrere Kontaktpunkte 27 für die Herstellung eines mechanischen Kontaktes mit entsprechenden Kontaktpunkten eines aufzusetzenden Eingabe- und Lesegerätes oder eines aufzusetzenden Kontaktelementes für die Herstellung einer Kabelverbindung mit einem Eingabe- und Lesegerät zugänglich sind.

Die Position der elektronischen Speichereinheit 22 ist sowohl durch ein passgerechtes Einpassen ihrer kreisförmig ausgebildeten Trägerplatte 35 in den zylindrischen Aufnahmeraum 21 als auch durch zwei diese Trägerplatte 35 stiftförmig durchdringende, innere Fortsätze 36, 37 des Innenverschlusses 16 gesichert.

Die elektronische Speichereinheit 22 ermöglicht die Aufnahme von umfangreichen Daten, einschliesslich von digitalen bildlichen Darstellungen, wie sie z.B. bei Dopingtests zusammengestellt werden können. Ausserdem können diese Daten die Speichereinheit 22 eindeutig dem Behälter 1 und seinem Inhalt zuordnen, indem an diesem und seiner Schutzkappe 4 von aussen zugängliche oder lesbare Beschriftungen 31, 32 oder Strichcodedarstellungen fest angebracht sind, die ausserdem als Bestandteil der Daten der elektronischen Speichereinheit 22 elektronisch gespeichert werden.

## Patentansprüche

1. Identifizierbarer Behälter für eine fälschungssichere Aufbewahrung von Substanzen oder Objekten, mit einer Füllöffnung (3) und einer dieser zugeordneten Verschlussvorrichtung (16,4), wobei die Verschlussvorrichtung Verriegelungsmittel (5,6,8) aufweist, durch die sie zerstörungsfrei unlösbar am Behälter (1) befestigt ist und mit einer ein- und auslesbaren elektronischen Speichereinheit (22), **dadurch gekennzeichnet, dass** die elektronische Speichereinheit (22) nur über mechanische Kontaktmittel (27) ein- und auslesbar ist und in der Verschlussvorrichtung (16) zugriffssicher eingeschlossen ist.

2. Behälter nach Anspruch 1, **dadurch gekennzeichnet, dass** die Verschlussvorrichtung mehrteilig zusammengesetzt ist, indem sie eine äussere Schutzkappe (4) aufweist, die einen inneren Dichtkörper (16) für den dichtenden Kontakt mit einer Füllöffnungsbegrenzung des Behälters (1) und die Verriegelungsmittel (5,6,8; 9,13) umschliesst.

3. Behälter nach Anspruch 2, **dadurch gekennzeichnet, dass** die Füllöffnung in einem ein Aussengewinde (2) aufweisenden Füllstutzen (3) vorgesehen ist und im Übergangsbereich (7) zwischen dem Behälter (1) und seinem Füllstutzen (3) an ihm Verriegelungsmittel in Form von Rastnocken (5,6) angeformt sind, die für den Eingriff von Verriegelungsmitteln (8,9,13) der Schutzkappe (4) bestimmt sind, wobei zwischen einem ein dazu passendes Innengewinde aufweisenden oberen Teil der Schutzkappe (4) und einem die Verriegelungsmittel (5,6; 8,9,13) einschliessenden unteren, sich über einen Schulterbereich (28) anschliessenden Kappenkragen (12) ein umlaufender Solltrennbereich (29) vorgesehen ist.

4. Behälter nach Anspruch 3, **dadurch gekennzeichnet, dass** der Solltrennbereich (29) durch eine auf der Innenseite der Schutzkappe vorgesehene Rille (2) gebildet ist.

5. Behälter nach einem der Ansprüche 2 bis 4, **dadurch gekennzeichnet, dass** die elektronische Speichereinheit (22) auf der dem Behälterinnenraum (26) abgekehrten Seite des inneren Dichtkörpers (16) angeordnet ist.

6. Behälter nach Anspruch 5, **dadurch gekennzeichnet, dass** der innere Dichtkörper (16) einen zylindrischen Aufnahmeraum (21) für das elektronische Speicherelement (22) aufweist.

7. Behälter nach Anspruch 5 oder 6, **dadurch gekennzeichnet, dass** die Kontur einer Trägerplatte (35) der elektronischen Speichereinheit (22) dem Aufnahmeraum (21) des inneren Dichtkörpers (16) formschlüssig angepasst ist.

8. Behälter nach einem der Ansprüche 5 bis 7, **dadurch gekennzeichnet, dass** die Position der elektronischen Speichereinheit (22) in dem Aufnahmeraum (21) des inneren Dichtkörpers (16) durch mindestens einen, ihre Trägerplatte (35) stiftförmig durchdringenden, inneren Fortsatz (37) des Innenverschlusses (16) gesichert ist.

9. Behälter nach einem der Ansprüche 5 bis 8, **dadurch gekennzeichnet, dass** an der im Aufnahmeraum (21) des inneren Dichtkörpers (16) freien Oberseite der elektronischen Speichereinheit (22) mehrere elektrische Kontaktpunkte (27) für die Verbindung mit einem Eingabe- und Lesegerät vorgesehen,sind.

10. Behälter nach einem der Ansprüche 1 bis 9, **dadurch gekennzeichnet, dass** er an ihm sichtbare Identifikationsmerkmale (31,32) aufweist und diese Identifikationsmerkmale zusätzlich in der elektronischen Speichereinheit (22) gespeichert sind.

11. Behälter nach einem der Ansprüche 1 bis 10, **gekennzeichnet durch** einen entfernbaren Abdeckring (15) der vor einer Benutzung des Behälters (1) dazu bestimmt ist, eine unbeabsichtigte Verriegelung seiner Schutzkappe (4) zu verhindern, indem er zwischen den Rastnocken (5,6) des Behälters (1) und den Verriegelungsmitteln (8,9,13) der Schutzkappe (4) angeordnet ist.

## Claims

1. Identifiable container for a counterfeit-proof storage of substances or objects, having a filling opening (3) and a closing device (16, 4) allocated to this, wherein the closing device has locking means (5, 6, 8) using which the closing device is fixed to the container (1) non-destructively and unreleasably, and having an electronic storage unit (22) that can be imported to and exported from, **characterised in that** the electronic storage device (22) is only able to be imported to and exported from via mechanical contact means (27) and is enclosed in the closing device (16) to prevent unauthorised access.

2. Container according to claim 1, **characterised in that** the closing device is composed of several parts **in that** it has an outer protective cover (4), which surrounds an inner sealing element (16) for the sealing contact with a filling opening limit of the container (1) and the locking means (5, 6, 8; 9, 13).

3. Container according to claim 2, **characterised in that** the filling opening is provided in a filling spout (3) having an external thread (2), and locking means are formed on it in the form of latch cams (5, 6) in the transition region (7) between the container (1) and its filling spout (3), said latch cams being designated for the engagement of locking means (8, 9, 13) of the protective cover (4), wherein a surrounding set separation region (29) is provided between an upper part of the protective cover (4) that as an inner thread that fits thereto and a lower cover collar (12) that encloses the locking means (5, 6, 8, 9, 13) and is connected via a shoulder region.

4. Container according to claim 3, **characterised in that** the set separation region (29) is formed by a groove (2) provided on the inner side of the protective cover.

5. Container according to one of claims 2 to 4, **characterised in that** the electronic storage unit (22) is arranged on the side of the inner sealing element (16) that is turned away from the inside of the container (26).

6. Container according to claim 5, **characterised in that** the inner sealing element (16) has a cylindrical receiving space (21) for the electronic storage element (22).

7. Container accordign to claim 5 or 6, **characterised in that** the contour of a carrier plate (35) of the electronic storage unit (22) is adapted positively to the receiving space (21) of the inner sealing element (16).

8. Container according to one of claims 5 to 7, **characterised in that** the position of the electronic storage unit (22) in the receiving space (21) of the inner sealing element (16) is secured by at least one inner extension (37) of the inner lock (16) that penetrates its carrier plate (35) in a pin-like manner.

9. Container according to one of claims 5 to 8, **characterised in that** several electric contact points (27) for the connection with an input and reader device are provided on the free upper sides of the electronic storage unit (22) in the receiving space (21) of the inner sealing element (16).

10. Container according to one of claims 1 to 9, **characterised in that** it has visible identification features (31, 32) on it and these identification features are additionally stored in the electronic storage unit (22).

11. Container according to one of claims 1 to 10, **characterised by** a removable cover ring (15) which is designated before using the container (2), to prevent an unintentional locking of its protective cover (4) in that it is arranged between the hatch cams (5, 6) of the container (1) and the locking means (8, 9, 13) of the protective cover (4).

## Revendications

1. Récipient identifiable pour une conservation infalsifiable de substances ou d'objets, avec une ouverture de remplissage (3) et un dispositif de fermeture (16, 4) associé à celle-ci, dans lequel le dispositif de fermeture présente des moyens de verrouillage (5, 6, 8) par lesquels il est fixé au récipient (1) de manière inséparable sans destruction et avec un module de mémoire électronique pouvant être mis en mémoire et lu (22), **caractérisé en ce que** le module de mémoire électronique (22) ne peut être mis en mémoire et lu que par le biais de moyens de contact mécaniques (27) et est enfermé de manière protégée contre un accès dans le dispositif de fermeture (16).

2. Récipient selon la revendication 1, **caractérisé en ce que** le dispositif de fermeture est composé de plusieurs parties **en ce qu'**il présente un capuchon de protection externe (4) qui entoure un corps d'étanchéité interne (16) pour le contact étanchéifiant avec une limite d'ouverture de remplissage du récipient (1) et les moyens de verrouillage (5, 6, 8 ; 9, 13).

3. Récipient selon la revendication 2, **caractérisé en ce que** l'ouverture de remplissage est prévue dans une tubulure de remplissage (3) présentant un filetage externe (2) et des moyens de verrouillage sous la forme de cames d'encliquetage (5, 6) qui sont destinés à l'engagement de moyens de verrouillage (8, 9, 13) du capuchon de protection (4) sont moulés sur la tubulure de remplissage (3) dans la région de transition (7) entre le récipient (1) et sa tubulure de remplissage (3), dans lequel une région périphérique destinée à la séparation (29) est prévue entre une partie supérieure du capuchon de protection (4) présentant un filetage interne s'adaptant à celui-ci et une collerette de capuchon inférieure (12) s'étendant sur une région d'épaulement (28), enfermant les moyens de verrouillage (5, 6 ; 8, 9, 13).

4. Récipient selon la revendication 3, caractérisé sen ce que la région destinée à la séparation (29) est formée par une rainure (2) prévue sur le côté interne du capuchon de protection.

5. Récipient selon l'une quelconque des revendications 2 à 4, **caractérisé en ce que** le module de mémoire électronique (22) est disposé sur le côté du corps d'étanchéité interne (16) écarté de l'espace interne du récipient (26).

6. Récipient selon la revendication 5, **caractérisé en ce que** le corps d'étanchéité interne (16) présente un espace de réception cylindrique (21) pour l'élément de mémoire électronique (22).

7. Récipient selon la revendication 5 ou 6, **caractérisé en ce que** le contour d'une plaque de support (35) du module de mémoire électronique (22) est adapté par conjugaison de formes à l'espace de réception (21) du corps d'étanchéité interne (16).

8. Récipient selon l'une quelconque des revendications 5 à 7, **caractérisé en ce que** la position du module de mémoire électronique (22) dans l'espace de réception (21) du corps d'étanchéité interne (16) est sécurisée par au moins un prolongement interne (37) de la fermeture interne (16) traversant sous forme de tige sa plaque de support (35).

9. Récipient selon l'une quelconque des revendications 5 à 8, **caractérisé en ce que** plusieurs points de contact électriques (27) pour la connexion à un appareil de saisie et de lecture sont prévus sur le côté supérieur libre dans l'espace de réception (21) du corps d'étanchéité interne (16) du module de mémoire électronique (22).

10. Récipient selon l'une quelconque des revendications 1 à 9, **caractérisé en ce qu'**ii présente des caractéristiques d'identification (31, 32) visibles sur lui et ces caractéristiques d'identification sont en outre mémorisées dans le module de mémoire électronique (22).

11. Récipient selon l'une quelconque des revendications 1 à 10, **caractérisé par** un anneau de recouvrement amovible (15) qui est destiné avant une utilisation du récipient (1) à empêcher un verrouillage involontaire de son capuchon de protection (4) en ce qu'il est disposé entre les cames d'encliquetage (5, 6) du récipient (1) et les moyens de verrouillage (8, 9, 13) du capuchon de protection (4).
